# EUROPEAN PATENT SPECIFICATION

(11) **EP 3 925 650 B1**
(45) Date of publication and mention of the grant of the patent: **04.11.2026**
(21) Application number: 21189923.2
(22) Date of filing: 12.08.2019
(51) Int. Cl.: A61M 5/28, A61M 5/31, A61M 5/34, A61J 1/06

(54) **BATCH OF GLASS SYRINGE BARRELS WITH INCREASED CONE BREAKING RESISTANCE**
CHARGE VON GLAS-SPRITZENZYLINDERN MIT ERHÖHTER BRUCHFESTIGKEIT DES KONUS
LOT DE CORPS DE SERINGUES EN VERRE PRÉSENTANT UNE RÉSISTANCE ACCRUE À LA RUPTURE DU CÔNE

(43) Date of publication of application: 22.12.2021
(62) Document number(s) of the earlier application(s) in accordance with Art. 76 EPC: 19191323.5 / 3 777 929
(73) Proprietor: SCHOTT Pharma AG & Co. KGaA, 55122 Mainz (DE); SCHOTT Pharma Schweiz AG, 9000 St. Gallen (CH)
(72) Inventor: Oberhänsli, Roman, 9000 St.Gallen (CH); Andreoli, Ivo, 9240 Uzwil (CH); Strassmann, Martha, 9127 St. Peterzell (CH); Langsdorf, Andreas, 55218 Ingelheim (DE); Thomas, Peter, 56073 Koblenz (DE)
(74) Representative: Herzog IP Patentanwalts GmbH

(56) References cited:
- DE-A1- 102009 031 689
- DE-A1- 102017 112 823
- JP-A- 2015 073 635
- US-A- 5 851 201
- ANONYMOUS: "ISO 11040-4 Third addition 2015-04.01 Prefilled syringes - Part 4: Glass barrels for injectables and sterilized subassembled syringes ready for filling", vol. ISO 11040-4:2015, 1 April 2015 (2015-04-01), pages 1 - 52, XP009513732, Retrieved from the Internet <URL:1077952576> [retrieved on 20190917]

## Description

The present invention relates to a plurality of glass syringe barrels with an at least partially conically shaped upper portion and having a longitudinal axis **L_{barrel},** the glass syringe barrel comprising a top end through which a liquid can be ejected and a bottom end into which a plunger stopper can be pushed, the glass syringe barrel comprising in a direction from the top end to the bottom i) a cone region, ii) a shoulder region and iii) a body region.

In order to guarantee a reliable use of pharmaceutical products, pre-filled one-time-use syringes are available commercially. They permit a rapid injection of the product that they contain after a comparatively simple manipulation or handling. This sort of pre-filled syringe has a syringe barrel made from glass or polymer with a syringe head formed on it, in which either a syringe needle is integrated or which has a Luer connecting cone of a conical connection, if necessary, a lockable cone connection (Luer lock). A grip plate is mounted on the other open end of the syringe barrel, either formed in one piece with it or put on it as a separate part. An elastomeric piston stopper is slidable through the open end of the syringe barrel. The piston stopper has a threaded blind hole, in which a piston rod with a threaded front end is screwable in various embodiments. The aforementioned single-use syringe, also called a ready-made syringe, with a syringe barrel made of glass, is described in Norm DIN ISO 11040, in which, for example, the syringe barrel is described in part 4. The elastomeric standard piston stopper and standard piston rod made of polymer with a cruciform cross section are described in part 5.

There are two varieties of Luer taper connections: locking and slipping. "Luer-Lock" style connectors are often generically referred to as "Luer-lock", and "Luer-slip" style connectors may be generically referred to as "slip tip". Luer-lock fittings are securely joined by means of a tabbed hub on the female fitting which screws into threads in a sleeve on the male fitting. Slip tip (Luer-slip) fittings simply conform to Luer taper dimensions and are pressed together and held by friction (they have no threads). A glass syringe designed for a Luer-lock style connector usually is characterized in that it comprises an upper portion that is at least partially conically shaped over which the Luer components are pushed, usually followed by a constriction region in which the diameter is reduced compared to the diameter of the cone region. The reduced diameter in the constriction region creates a notch around the conically shaped upper portion, which allows the Luer-lock adapter (which is a sleeve made of plastic that comprises the threads) to "snap in" and thus securely hold these components on the syringe.

In a glass syringe barrel comprising designed for a Luer-lock style connector or for a Luer-slip style connector the outer diameter of the conically upper portion is only about 4 mm with a wall thickness of the glass in the cone region of only about 1 to about 2 mm. Accordingly, such glass syringe barrels are very susceptible to mechanical stresses in the cone region, such as those mechanical stresses which occur when a Luer adapter is attached to the conically shaped upper portion, when a needle in a Luer adapter that is attached to the Luer cone is inserted into human or animal tissue as part of the intended use of such a syringe, or when such glass syringe barrels are transported in a packaging unit that contains large number of such syringes packaged in a confined space.

International Standard ISO 11040-4 (Third Edition 2015-04-01) discloses certain standards glass barrels for injectables and sterilized subassembled syringes ready for filling must fulfil.

DE 10 2017 112823 A1 discloses a syringe, comprising a syringe body including a cylindrical portion and a frustoconical portion adjoined to the cylindrical portion; and a Luer lock connector including a Luer collar with an inner thread and a Luer cone with a through-channel, the Luer collar and the Luer cone forming a bottom surface therebetween, the syringe body and the Luer lock connector being formed in one piece and comprising a cyclic olefin resin, the Luer collar including reinforcing ribs on its outer face.

US 5,851,201 discloses a syringe, comprising a hollow barrel with an open proximal end and a conical distal tip, a plunger assembly inserted into the open proximal end, the plunger assembly and the hollow barrel defining an internal variable volume chamber for retaining fluid, wherein the exterior surface of the tip is provided with a non-coated texture, such that the force required to disengage the tip from a conically-shaped female fitment when a fluid is present between the tip and the fitment is equal to or greater than the force required to disengage the tip and the fitment when the tip and the fitment are dry, and the force required to disengage the tip from the female fitment in the dry state does not exceed the force required to disengage an identically dimensioned coated tip from the female fitment in the dry state.

JP 2015/73635 A discloses a multilayer syringe barrel having excellent moldability. The multilayer syringe barrel includes a nozzle portion to which an injection needle can be connected, a cylindrical portion having a flange at an open end, and a shoulder connecting the nozzle portion and the cylindrical portion on the opposite side to the open end. The multilayer syringe barrel is characterized in that the angle formed between the central axis of the multilayer syringe barrel and the outer surface of the shoulder and the angle formed between the central axis and the inner surface of the shoulder are 100° to 140° and in that the thickness of the shoulder is on the shoulder side of the nozzle portion greater than the wall thickness.

DE 10 2009 031689 A1 discloses a method for forming glass tubes, in which a glass is heated to a temperature above the transformation temperature Tg and by pressing a mold with at least two mold jaws the tube surface is shaped, wherein the glass tube is rotated about its longitudinal axis and the mold jaws are rotated relative to the glass tube.

In general, the present disclosure describes a glass syringe barrel comprising an at least partially conically shaped upper portion, particularly a Luer cone designed for a Luer-lock style connector or for a Luer-slip style connector, which, compared to similar glass syringe barrels known from the prior art, has an improved resistance towards pressure that is applied onto the cone region of the glass syringe barrel. The present disclosure further describes a glass syringe barrel comprising an at least partially conically shaped upper portion, particularly a Luer designed for a Luer-lock style connector or for a Luer-slip style connector, which, compared to similar glass syringe barrels known from the prior art, has an improved resistance towards pressure that is applied onto the cone region of the glass syringe barrel and which has been prepared by a process as simple as possible, preferably from prefabricated glass tubing by shaping and separation.

The present disclosure further describes a process for the preparation of a glass syringe barrel comprising an at least partially conically shaped upper portion, particularly a Luer cone designed for a Luer-lock style connector or for a Luer-slip style connector, which, compared to similar glass syringe barrels known from the prior art, has an improved resistance towards pressure that is applied onto the cone region of the glass syringe from prefabricated glass tubing by shaping and separation, wherein no additional process steps such as a modification of the glass surface or the thickening of the glass in the cone- and/or shoulder region are required.

The claimed invention relates to a plurality of glass syringe barrels as defined in appended independent claim 1. The dependent claims provide further preferred embodiments.

A contribution to solving at least one of the objects according to the invention is made by an embodiment 1 of a plurality of glass syringe barrels, each glass syringe barrel having an at least partially conically shaped upper portion and each glass syringe barrel having a longitudinal axis **L_{barrel}** and comprising a top end through which a liquid can be ejected and a bottom end into which a plunger stopper can be pushed, each glass syringe barrel comprising in a direction from the top end to the bottom end:
i) a cone region having a first end that corresponds to the top end of the glass syringe barrel and a second end, wherein the cone region has a length **l₁** and an outer diameter **d₁** at the second end;
ii) a shoulder region having a first end that is adjacent to the second end of the cone region and a second end, wherein the outer contour of the shoulder region comprises a concave and substantially circular arc-shaped area **c₁** with an outer radius **r₁** beginning below the second end of the cone region and a convex and substantially circular arc-shaped area **c₂** with an outer radius **r₂** beginning above the second end of the shoulder region;
iii) a body region having a first end that is adjacent to the second end of the shoulder region and a second end that corresponds to the bottom end of the glass syringe barrel, wherein the body region has an outer diameter **d₂** and a glass thickness **n₂**;
wherein for the Luer cone breaking resistance the 1 % quantile of the glass syringe barrels contained in the plurality of glass syringe barrels is at least 95 N and most preferably at least 110N.

According to a further embodiment of the plurality of glass syringe barrels according to the present invention, for the Luer cone breaking resistance the 50 % quantile of the glass syringe barrels contained in the plurality of glass syringe barrels is at least 145 N, preferably at least 175 N, more preferably at least 205 N, even more preferably at least 230 N and most preferably at least 270 N.

A plurality of glass syringe barrels has a 1 % quantile of 50 N (this particular value not being according to the claimed invention but useful for understanding the applied calculation) for the Luer cone breaking resistance if at least 1 % of the glass syringe barrels have a Luer cone breaking resistance of 50 N or less and if at least 99 % of have a Luer cone breaking resistance of 50 N or more. A plurality of glass syringe barrels has a 50 % quantile of 145 N for the Luer cone breaking resistance if at least 50 % of the glass syringe barrels have a Luer cone breaking resistance of 145 N or less and if at least 50 % of have a Luer cone breaking resistance of 145 N or more.

*"A plurality of glass syringes"* in the sense of the present invention comprises at least 100 glass syringes even more preferably at least 200 glass syringes and most preferably at least 400 glass syringes. Furthermore, the plurality of glass syringes preferably has been collected arbitrarily and particularly has not been selected with regard to any property. For example, the plurality glass syringes may be the group of syringes which are packed together in a typical transport tray.

In an embodiment 2 of the plurality of glass syringe barrels according to the invention, the plurality of glass syringe barrels is designed according to its embodiment 1, wherein for at least 75 %, preferably for at least 85 %, more preferably for at least 95 % and most preferably for each glass syringe barrel contained in the plurality of glass syringe barrels the following condition are fulfilled:
if the glass syringe barrel is placed on a plane horizontal substrate with the outer surface of the body region on it, within any given cross-section of the glass syringe barrel that is located in a plane being centrically located in the glass syringe barrel and comprising the longitudinal axis **L_{barrel}** of the glass syringe barrel, **f(x)** defines the vertical distance between the substrate and the outer surface of the glass syringe barrel at a given position **x,** $k \left(x\right) = \left|f"\left(x\right)/{\left[1+f′{\left(x\right)}^{2}\right]}^{3 / 2}\right|$ defines the absolute value of the curvature of **f(x)** at a given position **x,** and
in the interval between **x** = **P₁** and **x** = **P₂** for any concave curvature in this interval the minimum value for (1/**k(x)**)/**n₂**² is at least 0.5 mm⁻¹, preferably at least 0.6 mm⁻¹, more preferably at least 0.75 mm⁻¹, even more preferably at least 0.85 mm⁻¹ and most preferably at least 1.0 mm⁻¹, wherein **P₁** defines the x-position at which the outer diameter of the glass syringe barrel is 0.95 × **d₂** and **P₂** is **P₁** + 3 ×**n₂.**

In an embodiment 3 of the plurality of glass syringe barrels according to the invention the plurality of glass syringe barrels is designed according to its embodiment 2, for at least 75 %, preferably for at least 85 %, more preferably for at least 95 % and most preferably for each glass syringe barrel contained in the plurality of glass syringe barrels in the interval between **x** = **P₁** and **x** = **P₂** the maximum value of the first derivative **f'(x)ₘₐₓ** of **f(x)** is less than 18, even more preferably less than 15, even more preferably less than 10 and most preferably less than 5.

In an embodiment 4 of the plurality of glass syringe barrels according to the invention, the plurality of glass syringe barrels is designed according to anyone of its embodiments 1 to 3, wherein for at least 75 %, preferably for at least 85 %, more preferably for at least 95 % and most preferably for each glass syringe barrel contained in the plurality of glass syringe barrels **r₂** is in the range from 1.4 to 2.9 mm, more preferably in the range from 1.6 to 2.7 mm, even more preferably in the range from 1.8 to 2.5 mm and most preferably in the range from 2.0 to 2.3 mm.

In an embodiment 5 of the plurality of glass syringe barrels according to the invention, the plurality of glass syringe barrels is designed according to anyone of its embodiments 1 to 4, wherein the glass syringe barrel further comprises a constriction region that is located between the cone region and the shoulder region, the constriction region having a first end that is adjacent to the second end of the cone region, a second end that is adjacent to the first end of shoulder region and an outer contour **c₃**, wherein the constriction region has a length **l₁**', a minimum outer diameter **d₁**' < **d₁** below the first end of the constriction region and an outer diameter **d₁**" at the second end of the constriction region.

In an embodiment 6 of the plurality of glass syringe barrels according to the invention, the plurality of glass syringe barrels is designed according to its embodiment 5, wherein the outer contour **c3** of the glass syringe barrel in the constriction region is conically shaped with **d₁**' < **d₁**" and wherein at the second end of the constriction region **c₃** merges into **c₁** without any offset.

In an embodiment 7 of the plurality of glass syringe barrels according to the invention, the plurality of glass syringe barrels is designed according to its embodiment 6, wherein in the constriction region a first line that runs parallel to the longitudinal axis **L_{barrel}** and a second line that runs parallel to **c₃** and that runs in the same plane as the first line include an angle **γ**, and wherein for at least 75 %, preferably for at least 85 %, more preferably for at least 95 % and most preferably for each glass syringe barrel contained in the plurality of glass syringe barrels **γ** is in the range from 0.3 to 2.5°, preferably in the range from 0.5 to 2.0° and most preferably in the range from 0.7 to 1.5°.

In an embodiment 8 of the plurality of glass syringe barrels according to the invention, the plurality of glass syringe barrels is designed according to anyone of its embodiments 1 to 7, wherein for at least 75 %, preferably for at least 85 %, more preferably for at least 95 % and most preferably for each glass syringe barrel contained in the plurality of glass syringe barrels **l₁** or, in case of a constriction region, the total length **l₁** + **l₁**', is in the range from 8 to 12 mm, preferably in the range from 8.25 to 10.5 mm, more preferably in the range from 8.5 to 10 mm, even more preferably in the range from 8.6 to 9.8 mm, even more preferably in the range from 8.7 to 9.6 mm and most preferably in the range from 9.4 to 9.6 mm.

In an embodiment 9 of the plurality of glass syringe barrels according to the invention, the plurality of glass syringe barrels is designed according to anyone of its embodiments 5 to 8, wherein for at least 75 %, preferably for at least 85 %, more preferably for at least 95 % and most preferably for each glass syringe barrel contained in the plurality of glass syringe barrels **l₁**' is in the range from 1 to 3 mm, preferably in the range from 1.1 to 2.5 mm, more preferably in the range from 1.2 to 2 mm even more preferably in the range from 1.3 to 1.8 mm and most preferably in the range from 1.4 to 1.6 mm.

In an embodiment 10 of the plurality of glass syringe barrels according to the invention, the plurality of glass syringe barrels is designed according to anyone of its embodiments 1 to 9, wherein for at least 75 %, preferably for at least 85 %, more preferably for at least 95 % and most preferably for each glass syringe barrel contained in the plurality of glass syringe barrels **d₁** is in the range from 4 to 4.8 mm, preferably in the range from 4.2 to 4.6 mm, more preferably in the range from 4.3 to 4.5 mm, even more preferably in the range from 4.35 to 4.45 mm and most preferably in the range from 4.40 to 4.42 mm. Preferably, **d₁** is the maximum outer diameter of the cone region. It is furthermore preferred that the second end of the cone region (or, if the cone region comprises a construction region, the second end of the constriction region) preferably corresponds to the point at which the outer diameter **d₁** exceeds a value of 4.4 mm

In an embodiment 11 of the plurality of glass syringe barrels according to the invention, the plurality of glass syringe barrels is designed according to anyone of its embodiments 1 to 10, wherein for at least 75 %, preferably for at least 85 %, more preferably for at least 95 % and most preferably for each glass syringe barrel contained in the plurality of glass syringe barrels **d₂** is in the range from 6 to 15 mm, preferably in the range from 6.5 to 14 mm, more preferably in the range from 7 to 13 mm, even more preferably in the range from 7.5 to 12 mm and most preferably in the range from 8 to 11 mm.

In an embodiment 12 of the plurality of glass syringe barrels according to the invention the plurality of glass syringe barrels is designed according to anyone of its embodiments 1 to 11, wherein the outer surface of glass syringe barrel in the cone region is roughened or is provided with a coating.

In an embodiment 13 of the plurality of glass syringe barrels according to the invention, the plurality of glass syringe barrels is designed according to anyone of its embodiments 5 to 12, wherein for at least 75 %, preferably for at least 85 %, more preferably for at least 95 % and most preferably for each glass syringe barrel contained in the plurality of glass syringe barrels **d₁'** is in the range from 3.4 to 5.2 mm, preferably in the range from 3.6 to 5 mm, more preferably in the range from 3.8 to 4.8 mm, even more preferably in the range from 4 to 4.6 mm and most preferably in the range from 4.2 to 4.4 mm.

In an embodiment 14 of the plurality of glass syringe barrels according to the invention, the plurality of glass syringe barrels is designed according to anyone of its embodiments 5 to 13, wherein in at least 75 %, preferably for at least 85 %, more preferably for at least 95 % and most preferably in each glass syringe barrel contained in the plurality of glass syringe barrels, the maximum thickness of the glass in the constriction region is in the range from 1.3 to 2.1 mm, preferably in the range from 1.4 to 2 mm, more preferably in the range from 1.5 to 1.9 mm, even more preferably in the range from 1.6 to 1.8 mm and most preferably in the range from 1.65 to 1.75 mm.

In an embodiment 15 of the plurality of glass syringe barrels according to the invention, the plurality of glass syringe barrels is designed according to anyone of its embodiments 5 to 14, wherein in at least 75 %, preferably for at least 85 %, more preferably for at least 95 % and most preferably in each glass syringe barrel contained in the plurality of glass syringe barrels the minimum thickness of the glass in the constriction region is in the range from 1.1 to 1.9 mm, preferably in the range from 1.2 to 1.8 mm, more preferably in the range from 1.3 to 1.7 mm, even more preferably in the range from 1.4 to 1.6 mm and most preferably in the range from 1.45 to 1.55 mm.

In an embodiment 16 of the plurality of glass syringe barrels according to the invention, the plurality of glass syringe barrels is designed according to anyone of its embodiments 1 to 15, wherein in at least 75 %, preferably for at least 85 %, more preferably for at least 95 % and most preferably in each glass syringe barrel contained in the plurality of glass syringe barrels the shoulder region is characterized by an outer shoulder angle **α** in the range from 6 to 28°, preferably in the range from 8 to 22°, more preferably in the range from 9 to 16°, even more preferably in the range from 10 to 15° and most preferably in the range from 11 to 14.

In an embodiment 17 of the plurality of glass syringe barrels according to the invention, the plurality of glass syringe barrels is designed according to anyone of its embodiments 1 to 16, wherein in at least 75 %, preferably for at least 85 %, more preferably for at least 95 % and most preferably in each glass syringe barrel contained in the plurality of glass syringe barrels the shoulder region is characterized by an inner shoulder angle **β** in the range from 22 to 50° preferably in the range from 25 to 40°, more preferably in the range from 26 to 35°, even more preferably in the range from 27 to 32° and most preferably in the range from 28 to 31°.

In an embodiment 18 of the plurality of glass syringe barrels according to the invention, the plurality of glass syringe barrels is designed according to anyone of its embodiments 1 to 17, wherein **nₛ** is the glass thickness in the shoulder region, measured at the point of the inner shoulder surface at which the surface for the first time forms an angle of 30° with longitudinal axis **L_{barrel},** and in at least 75 %, preferably for at least 85 %, more preferably for at least 95 % and most preferably in each glass syringe barrel contained in the plurality of glass syringe barrels **nₛ** is in the range from 1.2 to 2 mm, preferably in the range from 1.3 to 1.8 mm, more preferably in the range from 1.3 to 1.6 mm, even more preferably in the range from 1.35 to 1.5 mm and most preferably in the range from 1.35 to 1.45 mm.

In an embodiment 19 of the plurality of glass syringe barrels according to the invention, the plurality of glass syringe barrels is designed according to anyone of its embodiments 1 to 18, wherein **l₂** is the length of the body region and wherein in at least 75 %, preferably for at least 85 %, more preferably for at least 95 % and most preferably in each glass syringe barrel contained in the plurality of glass syringe barrels **l₂** is in the range from 30 to 60 mm, preferably in the range from 35 to 55 mm, more preferably in the range from 38 to 50 mm, even more preferably in the range from 39.5 to 45 mm and most preferably in the range from 39.5 to 41 mm.

In an embodiment 20 of the plurality of glass syringe barrels according to the invention, the plurality 1 or 2 of glass syringe barrels is designed according to anyone of its embodiments 1 to 19, wherein in at least 75 %, preferably for at least 85 %, more preferably for at least 95 % and most preferably in each glass syringe barrel contained in the plurality of glass syringe barrels **n₂** is in the range from 0.6 to 1.6 mm, preferably in the range from 0.7 to 1.5 mm, more preferably in the range from 0.8 to 1.4 mm, even more preferably in the range from 0.9 to 1.3 mm and most preferably in the range from 1 to 1.2 mm.

In an embodiment 21 of the plurality of glass syringe barrels according to the invention, the plurality of glass syringe barrels is designed according to anyone of its embodiments 1 to 20, wherein the glass syringe barrel has a nominal volume **V** and wherein in at least 75 %, preferably for at least 85 %, more preferably for at least 95 % and most preferably in each glass syringe barrel contained in the plurality of glass syringe barrels **V** in a range from 0.5 to 11 ml, preferably from 0.5 to 9 ml, more preferably from 0.5 to 7 ml, even more preferably from 0.5 to 5 ml, most preferably from 0.5 to 3 ml.

In an embodiment 22 of the plurality of glass syringe barrels according to the invention, the plurality of glass syringe barrels is designed according to anyone of its embodiments 1 to 21, wherein the glass syringe barrel has a length **l₃**, measured as the distance between the top end and the bottom end, and wherein in at least 75 %, preferably for at least 85 %, more preferably for at least 95 % and most preferably in each glass syringe barrel contained in the plurality of glass syringe barrels **l₃** in the range from 30 to 100 mm, preferably in the range from 35 to 95 mm, more preferably in the range from 40 to 90 mm, even more preferably in the range from 45 to 86 mm and most preferably in the range from 46 to 70 mm.

In an embodiment 24 of the plurality of glass syringe barrels according to the invention, the plurality of glass syringe barrels is designed according to anyone of its embodiments 1 to 23, wherein at least 75 %, preferably at least 85 %, more preferably at least 95 % and most preferably each glass syringe barrel contained in the plurality of glass syringe barrels is rotationsymmetric around the longitudinal axis **L_{barrel}** that runs parallel to the body region and that preferably goes through the center of the top end and the bottom end.

In an embodiment 24 of the plurality of glass syringe barrels according to the invention, the plurality of glass syringe barrels is designed according to anyone of its embodiments 1 to 23, wherein the glass syringe barrel is designed for a Luer-slip style connector and is sealed with a tip cap that is attached to the at least partially conically shaped upper portion.

In an embodiment 25 of the plurality of glass syringe barrels according to the invention, the plurality of glass syringe barrels is designed according to anyone of its embodiments 1 to 24, wherein the glass syringe barrel is designed for a Luer-lock style connector and comprises a Luer-lock adapter that is attached to the at least partially conically shaped upper portion.

In an embodiment 26 the plurality of glass syringe barrels according to the invention the plurality of glass syringe barrels is designed according to anyone of its embodiments 1 to 25, wherein a needle is attached to the at least partially conically shaped upper portion via a Luer connector and wherein the needle is sealed with a needle cap.

In an embodiment 28 of the plurality of glass syringe barrels according to the invention, the plurality of glass syringe barrels is designed according to anyone of its embodiments 1 to 27, wherein the syringe further comprises
iv) a flange region having a first end that is adjacent to the second end of the body region and a second end that comprises a finger flange and that corresponds to the bottom end of the glass syringe barrel, wherein the flange region has an outer diameter **d₃** > **d₂**.

In an embodiment 29 of the plurality of glass syringe barrels according to the invention, the plurality of glass syringe barrels is designed according to anyone of its embodiments 1 to 28, wherein the glass is of a type selected from the group consisting of is selected from the group consisting of a borosilicate glass, an aluminosilicate glass, soda lime glass and fused silica.

In an embodiment 30 of the plurality of glass syringe barrels according to the invention, the plurality of glass syringe barrels is designed according to anyone of its embodiments 1 to 29, wherein the glass syringe barrel comprises a coating that at least partially superimposes the interior surface of the body region.

Also disclosed is an embodiment 1 of a process or making an item, preferably a glass syringe barrel, comprising as process steps
I) loading a glass tube having a longitudinal axis **L_{tube}** (that corresponds to **L_{barrel}** in the finished glass syringe barrel), a first end and a further end into a machine, preferably a rotary machine, the glass tube having a wall thickness **n₂** and an outer diameter **d₂**;
II) heating the first end of the glass tube, while rotating around its longitudinal axis, to a temperature above its glass transition temperature, preferably above its softening temperature, with a heating element, preferably with a flame;
III) while the glass tube is rotating around its longitudinal axis, shaping the first end that has been heated using one or more molding tools that act on predetermined positions of the outer surface of the glass tube at the first end to form
   i) a cone region having a first end that corresponds to the top end of the glass syringe barrel and a second end, wherein the cone region has a length **l₁** and an outer diameter **d₁** at the second end;
   ii) a shoulder region having a first end that is adjacent to the second end of the cone region and a second end, wherein the outer contour of the shoulder region comprises a concave and substantially circular arc-shaped area **c₁** with an outer radius **r₁** beginning below the second end of the cone region and a convex and substantially circular arc-shaped area **c₂** with an outer radius **r₂** beginning above the second end of the shoulder region;
   iii) a body region having a first end that is adjacent to the second end of the shoulder region and a second end that preferably corresponds to the bottom end of the glass syringe barrel, wherein the body region has an outer diameter **d₂** and a glass thickness **n₂**;
   wherein shaping in process step III) is performed in such a way that **r₁** is in the range from 0.5 to 2.5 mm, more preferably in the range from 0.6 to 2 mm, even more preferably in the range from 0.7 to 1.8 mm and most preferably in the range from 0.8 to 1.5 mm.

In an embodiment 2 of the process 1, the process 1 is designed according to its embodiment 1, wherein shaping in process step III) is performed in such a way that an outer counter in the shoulder region is obtained that is characterized by the following features:
if the shaped glass tube is placed on a plane horizontal substrate with the outer surface of the body region on it, within any given cross-section of the glass syringe barrel that is located in a plane being centrically located in the glass syringe barrel and comprising the longitudinal axis **L_{tube}** of the shaped glass tube, **f(x)** defines the vertical distance between the substrate and the outer surface of the glass syringe barrel at a given position **x,** $k \left(x\right) = \left|f"\left(x\right)/{\left[1+f′{\left(x\right)}^{2}\right]}^{3 / 2}\right|$ defines the absolute value of the curvature of **f(x)** at a given position **x,** and
in the interval between **x** = **P₁** and **x** = **P₂** for any concave curvature in this interval the minimum value for (1/**k(x)**)/**n₂**² is at least 0.5 mm⁻¹, preferably at least 0.6 mm⁻¹, more preferably at least 0.75 mm⁻¹, even more preferably at least 0.85 mm⁻¹ and most preferably at least 1.0 mm⁻¹, wherein **P₁** defines the x-position at which the outer diameter of the glass syringe barrel is 0.95 × **d₂** and **P₂** is **P₁** + 3 × **n₂**.

In an embodiment 3 of the process 1, the process 1 is designed according to its embodiment 2, wherein shaping in process step III) is performed in such a way that in the interval between **x** = **P₁** and **x** = **P₂** the maximum value of the first derivative **f'(x)ₘₐₓ** of **f(x)** is less than 18, even more preferably less than 15, even more preferably less than 10 and most preferably less than 5.

In an embodiment 4 of the process 1, the process 1 is designed according to anyone of its embodiments 1 to 3, wherein shaping in process step III) is performed in such a way that **r₂** is in the range from 1.4 to 2.9 mm, more preferably in the range from 1.6 to 2.7 mm, even more preferably in the range from 1.8 to 2.5 mm and most preferably in the range from 2.0 to 2.3 mm.

In an embodiment 5 of the process 1, the process 1 is designed according to anyone of its embodiments 1 to 4, wherein **d₂** is in the range from 6 to 15 mm, preferably in the range from 6.5 to 14 mm, more preferably in the range from 7 to 13 mm, even more preferably in the range from 7.5 to 12 mm and most preferably in the range from 8 to 11 mm.

In an embodiment 6 of the process 1, the process 1 is designed according to anyone of its embodiments 1 to 5, wherein **n₂** is in the range from 0.6 to 1.6 mm, preferably in the range from 0.7 to 1.5 mm, more preferably in the range from 0.8 to 1.4 mm, even more preferably in the range from 0.9 to 1.3 mm and most preferably in the range from 1 to 1.2 mm.

In an embodiment 7 of the process 1, the process 1 is designed according to anyone of its embodiments 1 to 6, wherein shaping in process step III) is performed in such a way that a constriction region is formed that is located between the cone region and the shoulder region, the constriction region having a first end that is adjacent to the second end of the cone region, a second end that is adjacent to the first end of shoulder region and an outer contour **c₃**, wherein the constriction region has a length **l₁**', a minimum outer diameter **d₁**' < **d₁** below the first end of the constriction region and an outer diameter **d₁**" at the second end of the constriction region.

In an embodiment 8 of the process 1, the process 1 is designed according to its embodiment 7, wherein shaping in process step III) is performed in such a way that the outer contour **c3** of the glass syringe barrel in the constriction region is conically shaped with **d₁**' < **d₁**" and wherein at the second end of the constriction region **c₃** merges into **c₁** without any offset.

In an embodiment 9 of the process 1, the process 1 is designed according to its embodiment 8, wherein shaping in process step III) is performed in such a way that in the constriction region a first line that runs parallel to the longitudinal axis **L_{barrel}** and a second line that runs parallel to **c₃** and that runs in the same plane as the first line include an angle y, and wherein **γ** is in the range from 0.3 to 2.5°, preferably in the range from 0.5 to 2.0° and most preferably in the range from 0.7 to 1.5°.

In an embodiment 10 of the process 1, the process 1 is designed according to anyone of its embodiments 1 to 9, wherein shaping in process step III) is performed in such a way that **l₁** or, in case of a constriction region, the total length **l₁** + **l₁**', is in the range from 8 to 12 mm, preferably in the range from 8.75 to 10.5 mm, more preferably in the range from 8.5 to 10 mm, even more preferably in the range from 8.6 to 9.8 mm, even more preferably in the range from 8.7 to 9.6 and most preferably in the range from 9.4 to 9.6 mm.

In an embodiment 11 of the process 1, the process 1 is designed according to anyone of its embodiments 7 to 10, wherein shaping in process step III) is performed in such a way that **l₁**' is in the range from 1 to 3 mm, preferably in the range from 1.1 to 2.5 mm, more preferably in the range from 1.2 to 2 mm, even more preferably in the range from 1.3 to 1.8 mm and most preferably in the range from 1.4 to 1.6 mm.

In an embodiment 12 of the process 1, the process 1 is designed according to anyone of its embodiments 1 to 11, wherein shaping in process step III) is performed in such a way that **d₁** is in the range from 4 to 5 mm, preferably in the range from 4.1 to 4.8 mm, more preferably in the range from 4.2 to 4.6 mm, even more preferably in the range from 4.3 to 4.45 mm and most preferably in the range from 4.40 to 4.42 mm. Preferably, **d₁** is the maximum outer diameter of the cone region. It is furthermore preferred that the second end of the cone region preferably corresponds to the point at which the outer diameter **d₁** exceeds a value of 4.4 mm

In an embodiment 13 of the process 1, the process 1 is designed according to anyone of its embodiments 7 to 12, wherein shaping in process step III) is performed in such a way that s **d₁**' is in the range from 3.4 to 5.2 mm, preferably in the range from 3.6 to 5 mm, more preferably in the range from 3.8 to 4.8 mm, even more preferably in the range from 4 to 4.6 mm and most preferably in the range from 4.2 to 4.4 mm.

In an embodiment 14 of the process 1, the process 1 is designed according to anyone of its embodiments 7 to 13, wherein shaping in process step III) is performed in such a way that the maximum thickness of the glass in the constriction region is in the range from 1.3 to 2.1 mm, preferably in the range from 1.4 to 2 mm, more preferably in the range from 1.5 to 1.9 mm, even more preferably in the range from 1.6 to 1.8 mm and most preferably in the range from 1.65 to 1.75 mm.

In an embodiment 15 of the process 1, the process 1 is designed according to anyone of its embodiments 7 to 13, wherein shaping in process step III) is performed in such a way that the minimum thickness of the glass in the constriction region is in the range from 1.1 to 1.9 mm, preferably in the range from 1.2 to 1.8 mm, more preferably in the range from 1.3 to 1.7 mm, even more preferably in the range from 1.4 to 1.6 mm and most preferably in the range from 1.45 to 1.55 mm.

In an embodiment 16 of the process 1, the process 1 is designed according to anyone of its embodiments 1 to 15, wherein shaping in process step III) is performed in such a way that the shoulder region is characterized by an outer shoulder angle **α** in the range from 6 to 28°, preferably in the range from 8 to 22°, more preferably in the range from 9 to 16°, even more preferably in the range from 10 to 15° and most preferably in the range from 11 to 14°.

In an embodiment 17 of the process 1, the process 1 is designed according to anyone of its embodiments 1 to 16, wherein shaping in process step III) is performed in such a way that the shoulder region is characterized by an inner shoulder angle **β** in the range from 22 to 50° preferably in the range from 25 to 40°, more preferably in the range from 26 to 35°, even more preferably in the range from 27 to 32° and most preferably in the range from 28 to 31°.

In an embodiment 18 of the process 1, the process 1 is designed according to anyone of its embodiments 1 to 17, wherein shaping in process step III) is performed in such a way that, if **nₛ** is the glass thickness in the shoulder region, measured at the point of the inner shoulder surface at which the surface for the first time forms an angle of 30° with longitudinal axis **L_{barrel}**, **nₛ** is in the range from 1.2 to 2 mm, preferably in the range from 1.3 to 1.8 mm, more preferably in the range from 1.3 to 1.6 mm, even more preferably in the range from 1.35 to 1.5 mm and most preferably in the range from 1.35 to 1.45 mm.

In an embodiment 19 of the process 1, the process 1 is designed according to anyone of its embodiment 1 to 18, wherein the process further comprises the step of
IV) roughening the the outer surface of glass syringe barrel in the cone region or applying coating onto the outer surface of glass syringe barrel in the cone region.

In an embodiment 20 of the process 1, the process 1 is designed according to anyone of its embodiment 1 to 19, wherein the process further comprises the step of
V) cutting the glass tube, while rotating around its major axis, at a predetermined position above the first end to obtain a glass tube with a length **l_{tube}** comprising a first end that has been shaped by means of process steps I) to III) and second end, wherein cutting can be accomplished mechanically by means of a cutting device or can be accomplished thermally by means of a flame with which the glass is molten at the predetermined position and is then pulled apart;
VI) heating the second end of the glass tube, while rotating around its major axis, to a temperature above its glass transition temperature, preferably above its softening temperature, with a heating element, preferably with a flame;
VII) while the glass tube is rotating around its major axis, shaping the second end that has been heated using one or more molding tools that act on predetermined positions of the outer surface of the glass tube at the first end to form a finger flange.

In an embodiment 21 of the process 1, the process 1 is designed according to anyone of its embodiment 1 to 20, wherein the glass is of a type selected from the group consisting of is selected from the group consisting of a borosilicate glass, an aluminosilicate glass and fused silica.

In an embodiment 22 of the process 1, the process 1 is designed according to anyone of its embodiment 1 to 21, wherein the process further comprises the step of
VIII) superimposing at least a part of the interior surface of the body region with a coating.

Also disclosed is an embodiment 1 of a glass syringe barrel obtainable by the process 1 according to any of its embodiments 1 to 22. In a preferred embodiment of the glass syringe barrel, this glass syringe barrel shows the technical features of the glass syringe barrel contained in the plurality of glass syringe barrels of the invention according to any of its embodiments, respectively.

A contribution to solving at least one of the objects according to the invention is made by an embodiment 1 of a plurality of syringes comprising
A) a plurality of glass syringe barrels according to any of its preferred embodiments;
B) a plunger stopper that has been pushed into the bottom end of each of the glass syringe barrels contained in the plurality of glass syringe barrels.

In an embodiment 2 of the plurality of syringes according to the invention, the syringe further comprises
C) a pharmaceutical composition that is filled into at least a part of the inner volume of the body region.

### Glass syringe barrel

The glass syringe barrel may have any size or shape which the skilled person deems appropriate in the context of the invention. The first end of the glass syringe barrel an opening in the form of a channel located within the cone region through which a pharmaceutical composition that is contained in the glass syringe can be squeezed out of the glass syringe barrel and a second end (that is preferably provided with a finger flange) into which a plunger stopper can be pushed. Preferably, the glass syringe barrel is of a one-piece design that is prepared by providing a glass tube, preferably in form of a hollow cylinder, and forming the conically shaper upper portion, thereby obtaining the cone and the shoulder region (and optionally constriction region) and by forming a finger flange at the opposed end of the glass tube. A preferred glass syringe barrel is a prefilled glass syringe barrel that is filled with a pharmaceutical preparation. Preferably, the glass syringe barrel is rotationally symmetrical around the longitudinal axis **L_{barrel}** that preferably goes perpendicular through the centre of the body region.

### Glass

The glass of the glass syringe barrel be any type of glass and may consist of any material or combination of materials which the skilled person deems suitable in the context of the invention. Preferably, the glass is suitable for pharmaceutical packaging. Particularly preferable, the glass is of type I, more preferably type I b, in accordance with the definitions of glass types in section 3.2.1 of the European Pharmacopoeia, 7th edition from 2011. Additionally, or alternatively preferable to the preceding, the glass is selected from the group consisting of a borosilicate glass, an aluminosilicate glass, soda lime glass and fused silica; or a combination of at least two thereof. For the use in this document, an aluminosilicate glass is a glass which has a content of Al₂O₃ of more than 8 wt.-%, preferably more than 9 wt.-%, particularly preferable in a range from 9 to 20 wt.-%, in each case based on the total weight of the glass. A preferred aluminosilicate glass has a content of B₂O₃ of less than 8 wt.-%, preferably at maximum 7 wt.-%, particularly preferably in a range from 0 to 7 wt.-%, in each case based on the total weight of the glass. For the use in this document, a borosilicate glass is a glass which has a content of B₂O₃ of at least 1 wt.-%, preferably at least 2 wt.-%, more preferably at least 3 wt.-%, more preferably at least 4 wt.-%, even more preferably at least 5 wt.-%, particularly preferable in a range from 5 to 15 wt.-%, in each case based on the total weight of the glass. A preferred borosilicate glass has a content of Al₂O₃ of less than 7.5 wt.-%, preferably less than 6.5 wt.-%, particularly preferably in a range from 0 to 5.5 wt.-%, in each case based on the total weight of the glass. In a further aspect, the borosilicate glass has a content of Al₂O₃ in a range from 3 to 7.5 wt.-%, preferably in a range from 4 to 6 wt.-%, in each case based on the total weight of the glass.

A glass which is further preferred according to the invention is essentially free from B.

Therein, the wording "essentially free from B" refers to glasses which are free from B which has been added to the glass composition by purpose. This means that B may still be present as an impurity, but preferably at a proportion of not more than 0.1 wt.-%, more preferably not more than 0.05 wt.-%, in each case based on the weight of the glass.

### Shape of the outer contour in a defined area of the Luer cone

An important element of the glass syringe barrels contained in the plurality of glass syringe barrels according to the invention is the shape of the outer contour in the shoulder region that is formed in process step III) of the process 1, particularly the outer contour in the concave and substantially circular arc-shaped area of the shoulder region. The outer contour in the shoulder region is characterized in that **r₁**, i. e. the radius of the concave and substantially circular arc-shaped area **c₁** beginning below the second end of the cone region, is in the range from 0.5 to 2.5 mm. Furthermore, if the glass syringe barrel further comprises a constriction region that is localized between the cone region and the shoulder region and that is characterized by an outer contour **c₃**, it is also preferred that the glass syringe barrel in the constriction region is conically shaped in such a way that the diameter of the constriction region increases towards the shoulder region. In this context it is particularly preferred that the outer contour of the glass syringe barrel at the second end of the constriction region merges into the outer contour of the glass syringe barrel at the first end of the shoulder region without any offset.

### Pharmaceutical composition

In the context of the invention, every liquid pharmaceutical composition which the skilled person deems suitable to be used in a syringe comes into consideration. A pharmaceutical composition is a composition comprising at least one active ingredient. A preferred active ingredient is a vaccine. A further preferred pharmaceutical composition is a parenterialium, i.e. a composition which is intended to be administered via the parenteral route, which may be any route which is not enteral. Parenteral administration can be performed by injection, e.g. using a needle (usually a hypodermic needle) and a syringe.

### MEASUREMENT METHODS

The following measurement methods are to be used in the context of the invention. Unless otherwise specified, the measurements have to be carried out at an ambient temperature of 23°C, an ambient air pressure of 100 kPa (0.986 atm) and a relative atmospheric humidity of 50 %.

### Determination of the local curvature k(x)

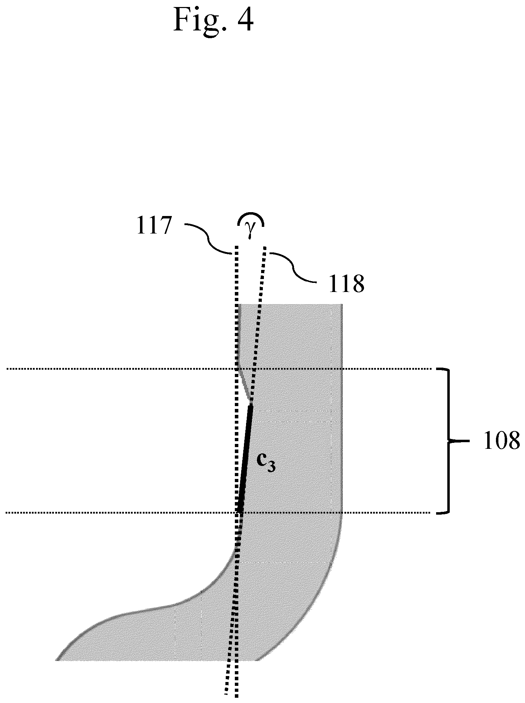

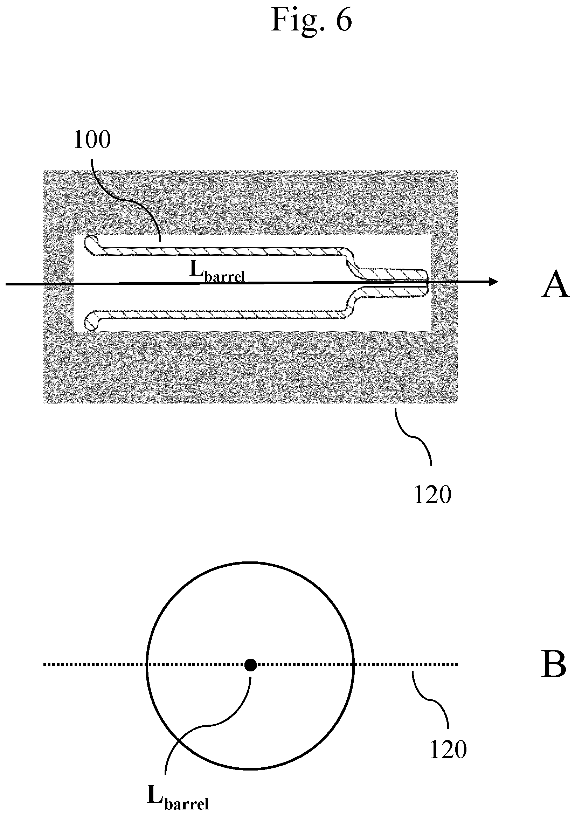

- The local curvature **k(x)** of the outer contour of the glass syringe barrel in the shoulder region defined by the function **f(x)** can be determined in a non-destructive manner using a profile projector. This approach is particularly suitable for glass syringe barrels that have been chemically and/or thermally tempered and that therefore cannot be easily sliced in half without the glass cracking or bursting. For determining the local curvature **k(x)** in a non-destructive manner the outer contour of the glass syringe barrels is visualized using a Mitutoyo PJ-3000 profile projector. The profile projector has a 10X magnification and is operated with transmitted light illumination. The barrels are placed in Hallbrite^{®} BHB (a butyloctyl salicylate obtainable from the Hallstar Company, Chicago, USA), which is filled into a glass bowl. Hallbrite^{®} BHB is used to visualize the outer contour of the barrel. It is ensured that the cross-section of the glass syringe barrel that is inspected in the profile projector corresponds to the plane that is centrically located in the glass syringe barrel and that comprises the longitudinal axis **L_{barrel}** of the glass syringe barrel, i. e. the axis that goes perpendicular through the barrel (see Figures 6A and 6B).
- To improve the measuring accuracy, the outer contour of the glass syringe barrel in the shoulder region can also be determined from a physical cross-sectional cut parallel along to the longitudinal axis **L_{barrel}** of the barrel (it is again ensured that the cross-section of the glass syringe barrel corresponds to the plane that is centrically located in the glass syringe barrel and that comprises the longitudinal axis glass syringe barrel as shown in Figure 4). For preparation without breakage, the barrel may be embedded into transparent 2-component epoxy resin, for example STRUERS GmbH, EpoFix Resin, or other suitable materials. After curing of the epoxy resin, a cross-sectional cut parallel along to the barrel axis can be achieved by machine-supported sawing, grinding and polishing. Geometrical features of the barrel can then be determined (measured) by means of non-distorting image capturing and geometrical analysis software tools.

The relevant outer contour of the outer surface of the glass syringe barrel in the shoulder region can be extracted and numerically approximated from the images obtained by means of the two approaches described above. For the extraction of the relevant contour of the outer surface, the images undergo the image processing steps implemented in Python [https://www.python.org/] based on the image processing library OpenCV [https://opencv.org/].

First, the images are denoised using a median filter. The denoised images are then processed with an edge detection algorithm based on a Sobel filter, in which the contours are identified by thresholding the gradient image. For the calculation of slopes and curvatures, the extracted contours are numerically approximated by a univariate spline of order 5. The radii of curvature **R(x)** are then given by the formula $R = \frac{{\left(1+{\left(\frac{dx}{dy}\right)}^{2}\right)}^{\frac{3}{2}}}{\frac{{d}^{2} x}{{dy}^{2}}}$ wherein **R(x)** = 1/**k(x).**

### Determination of r₁ and r₂

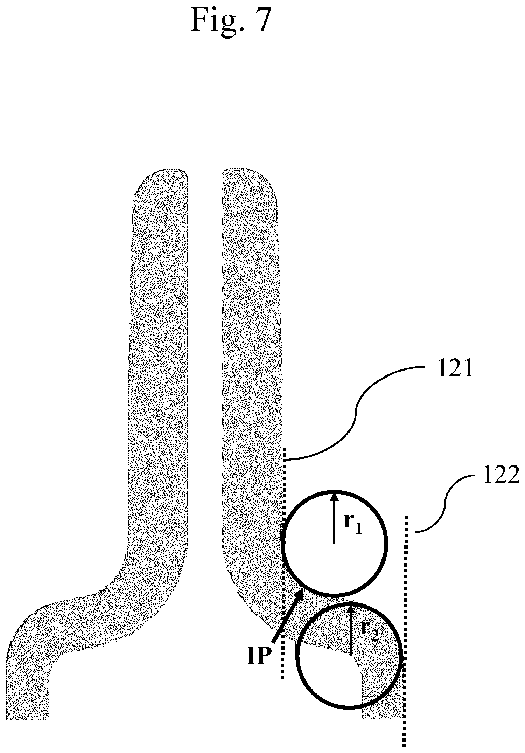

In the images obtained by means of the two approaches described above **r₁** can be determined geometrically by applying a bevel circle to the first inflection point **IP** (i. e. the first one that appears when entering the outer contour of the shoulder region from the top of the glass syringe barrel) of function **f(x)** (i. e. the first point at which the condition **f"(x)** = 0 is fulfilled) and to a straight line extending the outer contour of the cone region, as shown in Figure 7. **r₂** can also be determined geometrically by applying a circle adjacent to a straight line extending the outer contour of the body region and by increasing the diameter of the circle step by step until a maximum overlap between the outer contour of the shoulder region and a segment of the circle is reached (see again Figure 7). The radius of the thus obtained circle is **r₂**.

### Determination of nₛ

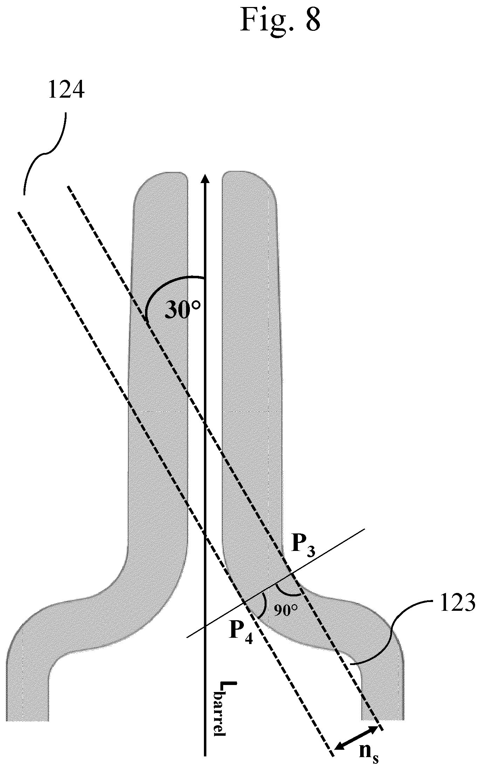

In the images obtained by means of the two approaches described above the thickness of the glass in the shoulder region **nₛ** is measured at that point **P₃** of shoulder region at which a tangent at the outer surface for the first time forms an angle of 30° to the syringe axis **L_{barrel}** as this is shown in Figure 8. **nₛ** is determined in a direction perpendicular to that line. For that purpose, a line is drawn perpendicular to that tangent in **P₃**. The point at which this perpendicular line crosses the inner surface is named **P₄**. A third line is drawn that runs parallel to tangent and that goes through **P₄**. **nₛ** corresponds to the distance between these two parallel lines.

### Determination of α and β

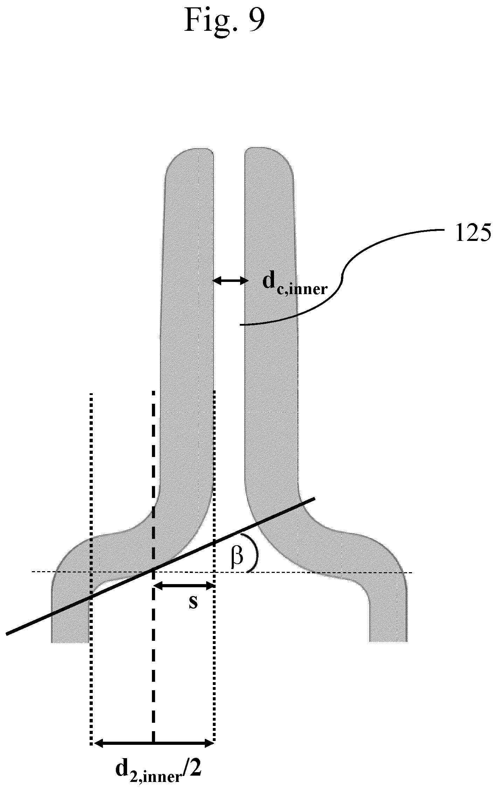

In the images obtained by means of the two approaches described above the inner shoulder angle **β** is measured at that point of the inner shoulder whose distance to the syringe axis **L_{barrel}** is [**d_{c,inner}** + **d_{2.inner}**)/4 as this is shown in Figure 9. **d_{c,inner}** corresponds to the inner diameter of the channel at the top end of the syringe barrel and **d_{2,inner}** corresponds to the inner diameter of the body region. **α** is determined using an offline camera. A line is drawn on the longest straight part between the two radii. The angle is then measured by applying a horizontal line.

### Determination of the cone breaking force

The cone breaking force is determined according to ISO 11040-4: 2015 C.2, wherein the following parameters have been selected:

| Method | Measurement system | Load cell | Test speed | sampling rate | Distance from tip end | Closure |
|---|---|---|---|---|---|---|
| selected values | tensile and compression testing machine | ≤ 2 kN | 25m / min | ≥ 100 Hz | 2-3 mm | LLA only |

### EXAMPLE

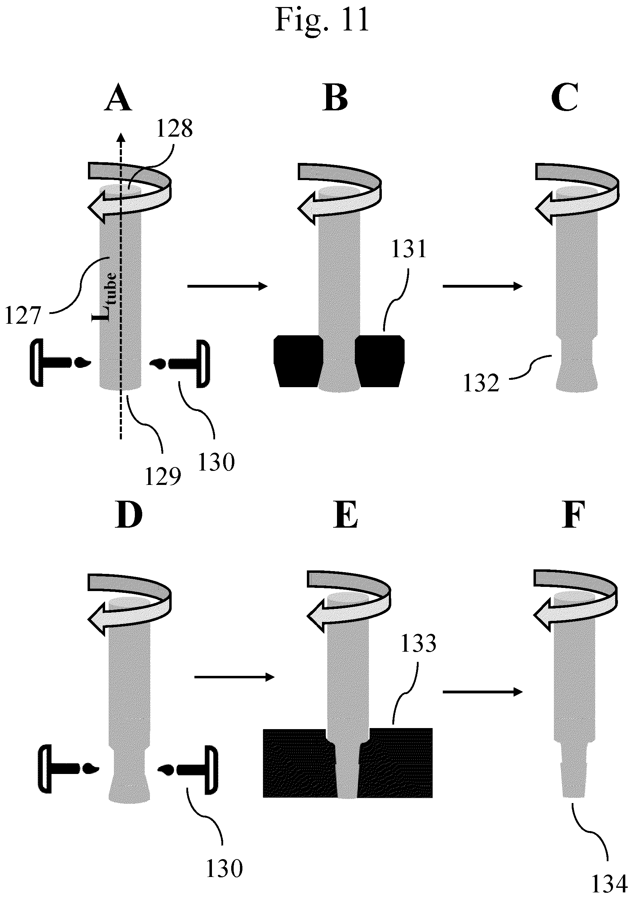

A glass tube having an outer diameter **d₂** of 10.85 mm and a wall thickness **n₂** of 1.1 mm made of borosilicate glass is loaded into the head of a rotary machine. While rotating around its longitudinal axis **L_{tube}** one end of the glass tube (i. e. at the end of which the Luer cone will be localized) is heated to its softening point with flames (see Fig. 11A). While the glass tube is rotating around its longitudinal axis the end that has been heated is shaped using molding tools that act on predetermined positions of the outer surface of the glass tube at the first end to form the cone region that also comprises a constriction region and the shoulder region. In a first step a first molding tool is pressed against the outer surface of the glass tube at the first end to initiate the formation of the constriction region (see Fig. 11B). The first end is then further heated (see Fig. 11D) and a set of second molding tools is then pressed against the preformed outer surface of the glass tube at the first end to obtain the final shape of the cone region, the constriction region and the shoulder region (see Figure 11E). The shape of the further molding tools as well as the angle in which these tools are pressed against the molten cone region ensure that the minimum radius of curvature between points **P₁** and **P₂** is maintained in the desired range.

In a further process step the glass tube, while rotating around its longitudinal, is cut at a predetermined position above the first end to obtain a glass tube with a length **l_{tube}** comprising a first end that is cone shaped and second end. The glass tube is then heated at the second end, while rotating around its longitudinal, to a temperature above its glass transition temperature with a flame. While the glass tube is still rotating around its longitudinal, the second end is then shaped for the formation of a flinger flange by pressing appropriate molding tools against the outer surface of the glass tube at the second end.

500 glass syringe barrels have been prepared in the rotary machine. The outer contour in the shoulder region of one of the glass syringe barrels corresponds to the outer contour of the shoulder region in glass syringe barrels known in the prior art (Comparative Example).

| | Comparative Example 1 | Example 1 |
|---|---|---|
| **r₁** [mm] | 0.4 | 1.5 |
| **r₂** [mm] | 1.8 | 2.3 |
| (1/**k(x)**)/**n₂**² [mm⁻¹] | 0.17 | 0.74 |
| **f'(x)ₘₐₓ** | 19 | 4.45 |
| Tip resistance Mean [%] | 100 | 183 |
| Tip resistance Median [N] | 142 | 275 |
| Tip resistance Median [%] | 100 | 194 |
| 1 % Quantile [N] | 48.3 | 116.5 |

Unless otherwise specified in the description or the particular Figure:
- Figure 1: shows a cross-sectional view of a syringe comprising a glass syringe barrel 100 into which a plunger stopper 104 has been introduced;
- Figure 2: shows a cross-sectional enlarged view of the top end of a glass syringe barrel 100 that comprises a conically shaped upper portion 101 in the form of a Luer cone;
- Figure 3: shows a cross-sectional enlarged view of the top end 102 of a further glass syringe barrel 100 that comprises a conically shaped upper portion 101 in the form of a Luer cone, wherein the glass syringe barrel further comprises a constriction region 108 that is located between the cone region 105 and the should region 111.;
- Figure 4: shows the determination of angle γ in the constriction region 108;
- Figure 5A: shows a cross-sectional enlarged view of the top end 102 of the glass syringe barrel 100 shown in Figure 3, wherein in contrast to Figure 3 the glass syringe barrel 100 with the outer surface of the body region 114 is laying on a sub- strate 119;
- Figure 5B: shows function **f(x)** by means of which the curvature **k(x)** of the outer contour of the glass syringe barrel in the shoulder region 111 can be determined;
- Figur 6A: shows in a side view the localization of plane 120 that is used to determine the local curvature of function **f(x)** within the range from **P₁** to **P₂**;
- Figure 6B: shows in a top view the localization of plane 120 that is used to determine the local curvature of function **f(x)** within the range from **P₁** to **P₂**;
- Figure 7: shows the determination of **r₁**;
- Figure 8: shows the determination of **nₛ**;
- Figure 9: shows the determination of **β**;
- Figure 10: shows a glass syringe barrel 100 according to the present invention with a tip cap 117 attached to the conically shaped upper portion 101;
- Figures 11A-F: illustrate steps I), II) and III) of process 1 for the preparation of a glass syringe barrel 100.

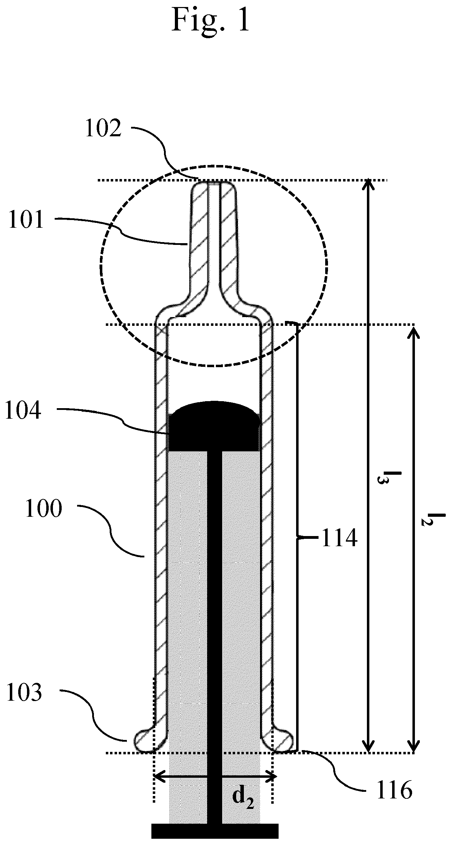

Figure 1 shows a cross-sectional view of a syringe comprising a glass syringe barrel 100 having the length **l₃** and an outer diameter **d₂** in the body region 114 into view a plunger stopper 104 has been introduced. As can be seen in Figure 1, the syringe barrel 100 comprises a top end 102 with a conically shaper upper portion 102 and a bottom end 103 into which the plunger stopper 104 has been introduced.

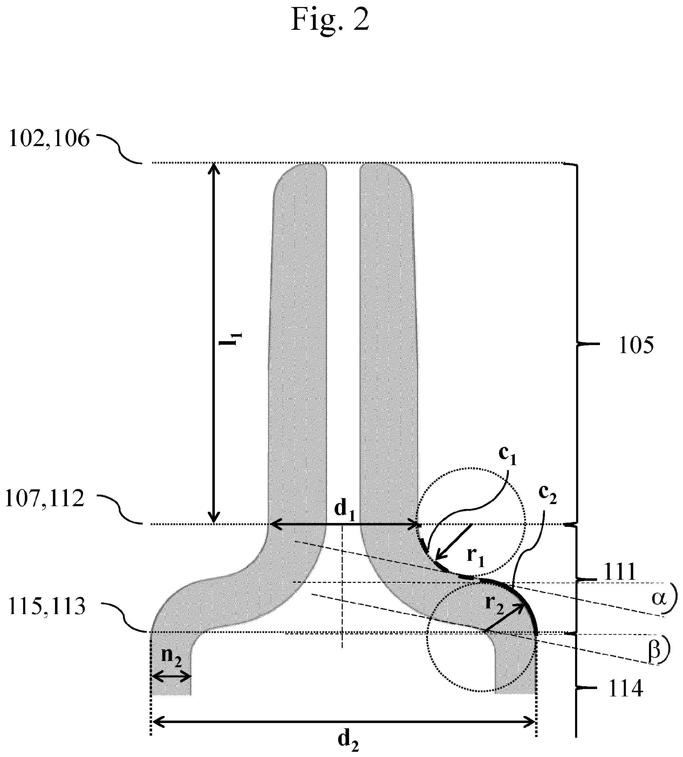

Figure 2 shows a cross-sectional enlarged view of the top end 102 of the glass syringe barrel 100 shown in Figure 1 that comprises the conically shaped upper portion 101 (the section of the glass syringe barrel 100 that is shown in Figure 2 corresponds to the area that is encompassed by the dotted circle in Figure 1). As can be seen in Figure 2, the conically shaped upper portion 101 of the glass syringe barrel 100 is in the form of a Luer-slip style connector and comprises in the embodiment shown in Figure 2 a cone region 105 having a first end 106 that corresponds to the top end 102 of the glass syringe barrel 100 and a second end 107, wherein the cone region 105 has a length **l₁** and an outer diameter **d₁** at the second end 107. Adjacent to the cone region 105 is a shoulder region 111 having a first end 112 that is adjacent to the second end 107 of the cone region 105 and a second end 113, wherein the outer contour of the shoulder region 111 comprises a concave and substantially circular arc-shaped area **c₂** (see the dashed and bold marked line in the upper part of the right side of the shoulder region) with an outer radius **r₁** beginning below the second end 110 of the constriction region 108 and a convex and substantially circular arc-shaped area **c₃** (see the continuous and bold marked line in the upper part of the right side of the shoulder region) with an outer radius **r₂** beginning above the second end 113. As also shown in Figure 2 the shoulder region 111 is characterized by an outer shoulder angle **α** and an inner shoulder angle **β.** Adjacent to the should region 111 is a body region 114 (see also Figure 1) into which the syringe plunger 104 can be pushed, having a first end 115 that is adjacent to the second end 113 of the shoulder region 111 and a second end 116 that corresponds to the bottom end 103 of the glass syringe barrel 100, wherein the thickness of the glass in the body region 114 is **n₂**. The diameter **d₂** of the body region 114 corresponds to the diameter of the glass tube 126 hat us used to manufacture the glass syringe barrel 100 according to the present invention (see Figure 11).

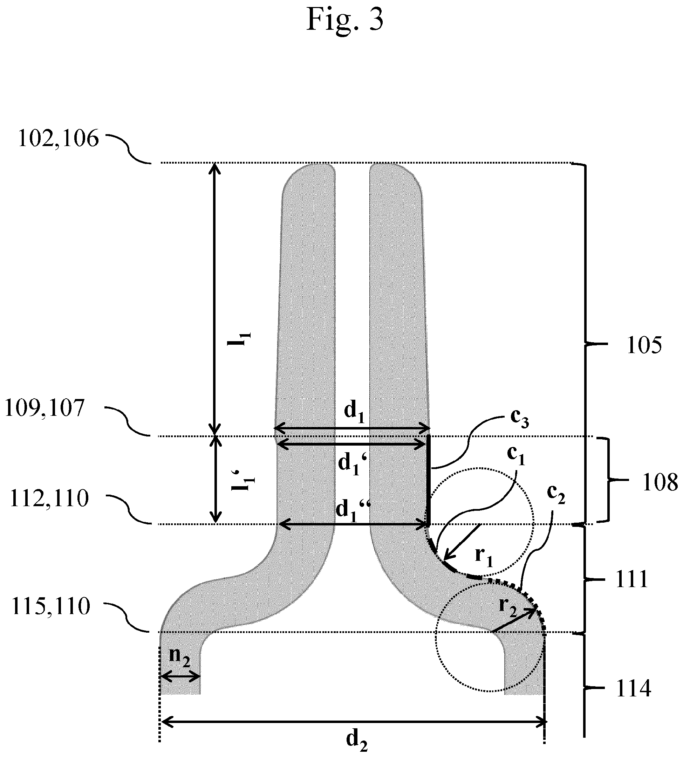

Figure 3 shows a cross-sectional enlarged view of the top end 102 of a further glass syringe barrel 100 that comprises a conically shaped upper portion 101 in the form of a Luer cone, wherein the glass syringe barrel further comprises a constriction region 108 that is located between the cone region 105 and the should region 111. The constriction region 108 comprises a first end 109 that is adjacent to the second end 107 of the cone region 105, a second end 110 that is adjacent to the first end 112 of shoulder region 111 and an outer contour **c₃**, wherein the constriction region 108 has a length **l₁**', a minimum outer diameter **d₁**' < **d₁** below the first end 109 of the constriction region 108 and an outer diameter **d₁**" at the second end 110 of the constriction region 108. In this context it is furthermore preferred that the outer contour **c3** of the glass syringe barrel in the constriction region is conically shaped with **d₁**' < **d₁**" and wherein at the second end of the constriction region **c₃** merges into **c₁** without any offset. It is also preferred that in the constriction region 108 a first line 117 that runs parallel to the longitudinal axis **L_{barrel}** and a second line 118 that runs parallel to **c₃** and that runs in the same plane as the first line 117 include an angle **γ,** wherein **γ** is in the range from 1 to 3°. Figure 4 shows how to determine the angle **γ** in the constriction region 108, this angle defining the extend of the conical shape of the constriction region.

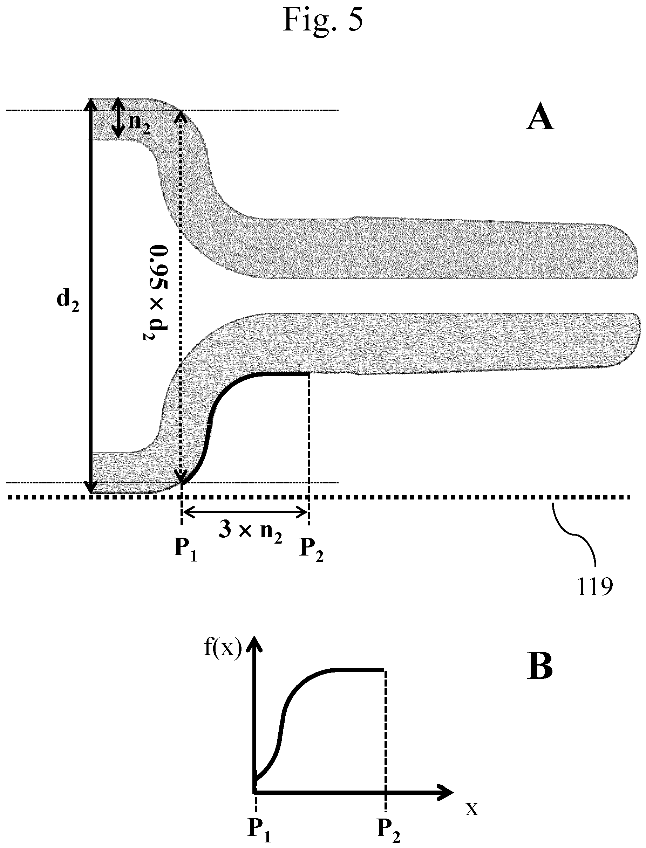

The glass syringe barrel 100 is characterized by a well-defined outer contour in the transition region between the shoulder region 111 and, if present, of the constriction region 108 as well. If the glass syringe barrel 100 is placed on a plane horizontal substrate 119 with the outer surface of the body region 114 on it as shown in Figure 5A, function **f(x)** defines the vertical distance between the substrate 119 and the outer surface of the glass syringe barrel 100 at a given position **x** and thus the outer contour of the glass syringe barrel 100 in that area (see Figure 5B). From that function the curvature **k(x)** at a given position **x** of the outer contour can be calculated as $k \left(x\right) = \left|f"\left(x\right)/\left.{\left[1+f′{\left(x\right)}^{2}\right]}^{3 / 2}\right).\right.$

The glass syringe barrel is now characterized in that interval between **x** = **P₁** and **x** = **P₂** for any concave curvature in this interval the minimum value for (1/**k(x)**)/**n₂**² is at least 0.5 mm⁻¹, wherein **P₁** defines the x-position at which the outer diameter of the glass syringe barrel 100 is 0.95 × **d₂** and **P₂** is **P₁** + 3 × **n₂** (see Figure 5A).

Figures 6A and 6B show in a side view and in a top view the localization of plane 120 in the glass syringe barrel 100 that is used to determine the local curvature of function **f(x)** within the range from **P₁** to **P₂** by means of the approach that is shown in Figures 5A and 5B. Plane 120 corresponds to the plane that is centrically located in the glass syringe barrel 100 and that comprises the longitudinal axis **L_{barrel}** of the glass syringe barrel 100.

Figure 7 shows how to determine the outer radius **r₁** of the concave and substantially circular arc-shaped area **c₁** of the shoulder region 111. **r₁** can be determined geometrically in cross-sectional images of the glass syringe barrel 100 by applying a bevel circle to the first inflection point **IP** (i. e. the first one that appears when entering the outer contour of the shoulder region 111 from the top end 102 of the glass syringe barrel 100) of function **f(x)** (i. e. the first point at which the condition **f"(x)** = 0 is fulfilled) and to a straight line 121 extending the outer contour of the cone region 105. **r₂** can be determined geometrically by applying a circle adjacent to a straight line 122 extending the outer contour of the body region 114 and by increasing the diameter of the circle until a maximum overlap between the outer contour of the shoulder region 111 and a segment of the arc is reached. The radius of the thus obtained circle is r₂.

Figure 8 shows how to determine the thickness **nₛ** of the glass in the shoulder region 111. **nₛ** is measured at that point P₃ of shoulder region at which a tangent at the outer surface for the first time forms an angle of 30° to the syringe axis **L_{barrel}** as this is shown in Figure 8. **nₛ** is determined in a direction perpendicular to that line. For that purpose, a line is drawn perpendicular to that tangent in P₃. The point at which this perpendicular line crosses the inner surface is named P₄. A third line is drawn that runs parallel to tangent and that goes through P₄. **nₛ** corresponds to the distance between these two parallel lines.

Figure 9 shows how to determine the inner shoulder angle **β**. **β** is measured at that point of the inner shoulder whose distance to the syringe axis **L_{barrel}** is **[d_{c,inner}** + **d_{2.inner})**/4. **d_{c,inner}** corresponds to the inner diameter of the channel 125 at the top end 102 of the glass syringe barrel 100 and **d_{2,inner}** corresponds to the inner diameter of the body region 114.

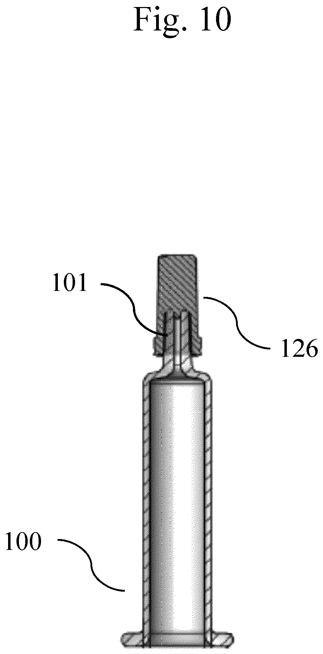

Figure 10 shows a glass syringe barrel 100 with a tip cap 126 attached to the conically shaped upper portion 101.

Figures 11A-F illustrate steps I), II) and III) of process 1 for the preparation of a glass syringe barrel 100. In process step I) a glass tube 127 having a longitudinal axis **L_{tube},** a first end 129 and a further end 128 is loaded into a machine, preferably a rotary machine, the glass tube 127 having a wall thickness **n₂** and an outer diameter **d₂.** In process step II) the glass tube 127, while rotating around its longitudinal axis, is heated to a temperature above its glass transition temperature, preferably above its softening temperature, with a heating element 130 (indicated by the candle flames shown on the left in Figure 11A), preferably with a flame 130. In process step III), while the glass tube 127 is still rotating around its longitudinal axis, the first end 129 that has been heated is shaped by using molding tools 131 that act on predetermined positions of the outer surface of the glass tube 127 at the first end 129 to form a conically shaped upper portion 101 as shown in Figures 11B and 11E. In the process shown in Figure 11 the conically shaped upper portion 101 is formed in two steps: in a first step using a first set of molding tools 131 a Luer end precursor 132 is formed that does not have the final shape of the conically shaped upper portion 101 according to the present invention (see Figures 11B and 11C). The Luer cone precursor 132 is then again heated as shown in Figure 11D and is then finally shaped using a second set of molding tools 133 as shown in Figure 11E to obtain a glass tube 127 with finished conically shaped upper portion 101 (see Figure 11E). The shape of the molding tools 131,133 that are used in this shaping process as well as the extent to which they are pressed against the molten region of the glass tube 127 have to be adopted to ensure that the desired geometry particularly in the transition state between the shoulder region 111 and the cone region 105 (or, if present, the constriction region 108) is obtained.

In a further process step V) the glass tube with finished conically shaped upper portion 101, while rotating around its longitudinal axis, is cut at a predetermined position above the first end 129 to obtain a glass tube with a length **l_{tube}** comprising a first end 128 that has been shaped by means of process steps I) to III) and second end. In a further process step VI) the second end of the glass tube, while rotating around its longitudinal axis, is heated to a temperature above its glass transition temperature, preferably above its softening temperature, with a heating element, preferably with a flame. In a further process step VII), while the glass tube is rotating around its longitudinal axis, the second end that has been heated using is shaped using one or more molding tools that act on predetermined positions of the outer surface of the glass tube at the first end to, for example, form a finger flange (process steps V) to VII) are not shown in Figure 11).

### LIST OF REFERENCE NUMERALS

- **100**: glass syringe barrel
- **101**: conically shaped upper portion
- **102**: top end
- **103**: bottom end
- **104**: plunger stopper
- **105**: cone region
- **106**: first end of the cone region 105 (= top end 102)
- **107**: second end of the cone region 105 (= first end 112 of the shoulder region 111 or first end 109 of the constriction region 108)
- **111**: shoulder region
- **112**: first end of the shoulder region 111 (= second end 107 of the cone region 105 or second end 110 of the constriction region 108)
- **113**: second end of the shoulder region 111 (= first end 115 of the body region 114)
- **114**: body region
- **115**: first end of the body region 114 (= second end 113 of the shoulder region 111)
- **116**: second end of the body region 114 (= bottom end 103)
- **108**: constriction region
- **109**: first end of the constriction region 108 (= the second end 107 of the cone region 105)
- **110**: second end of the constriction region 108 (= first end 112 of the should region 111)
- **117**: first line
- **118**: second line
- **119**: plane and horizontal substrate
- **120**: cross-sectional plane in the middle of the glass syringe barrel 100
- **121**: elongation of the outer contour of the cone region 105
- **122**: elongation of the outer contour of the body region 114
- **123**: tangent at the outer surface of the shoulder region 111 that includes an angle of 30° with **l_{barrel}**
- **124**: tangent at the inner surface of the shoulder region 111 that includes an angle of 30° with **l_{barrel}**
- **125**: channel at the top end 102
- **126**: tip cap
- **127**: glass tube
- **128**: further end of the glass tube
- **129**: first end of the glass tube
- **130**: heating element
- **131**: first set of forming tools
- **132**: Luer end precursor
- **133**: second set of molding tools
- **134**: glass tube with finished Luer end

## Claims

1. A plurality of glass syringe barrels (100) that comprises at least 100 glass syringe barrels (100), each glass syringe barrel (100) having an at least partially conically shaped upper portion (101) and each glass syringe barrel (100) having a longitudinal axis **L_{barrel}** and comprising a top end (102) through which a liquid can be ejected and a bottom end (103) into which a plunger stopper (104) can be pushed, each glass syringe barrel (100) comprising in a direction from the top end (102) to the bottom end (103):
i) a cone region (105) having a first end (106) that corresponds to the top end (102) of the glass syringe barrel (100) and a second end (107), wherein the cone region (105) has a length **l₁** and an outer diameter **d₁** at the second end (107);
ii) a shoulder region (111) having a first end (112) that is adjacent to the second end (107) of the cone region (105) and a second end (113), wherein the outer contour of the shoulder region (111) comprises a concave and substantially circular arc-shaped area **c₁** with an outer radius **r₁** beginning below the second end (107) of the cone region (105) and a convex and substantially circular arc-shaped area **c₂** with an outer radius **r₂** beginning above the second end (113) of the shoulder region (111);
iii) a body region (114) having a first end (115) that is adjacent to the second end (113) of the shoulder region (111) and a second end (116) that corresponds to the bottom end (103) of the glass syringe barrel (100), wherein the body region (114) has an outer diameter **d₂** and a glass thickness **n₂**;
wherein for the Luer cone breaking resistance the 1 % quantile of the glass syringe barrels contained in the plurality of glass syringe barrels (100) is at least 95 N;
wherein for at least 75 % of the glass syringe barrels (100) contained in the plurality of glass syringe barrels (100) **r₁** is in the range from 0.5 to 2.5 mm;
wherein for at least 75 % of the glass syringe barrels (100) contained in the plurality of glass syringe barrels (100) **r₂** is in the range from 1.4 to 2.9 mm.

2. The plurality of glass syringe barrels (100) according to claim 1, wherein the plurality of glass syringe barrels (100) comprises at least 200 glass syringes barrels (100).

3. The plurality of glass syringe barrels (100) according to claim 1 or 2, wherein for at least 75 % of the glass syringe barrels (100) contained in the plurality of glass syringe barrels (100) **r₁** is in the range from 0.6 to 2 mm.

4. The plurality of glass syringe barrels (100) according to anyone of claims 1 to 3, wherein for at least 75 % of the glass syringe barrels (100) contained in the plurality of glass syringe barrels (100) the following conditions are fulfilled:
if the glass syringe barrel (100) is placed on a plane horizontal substrate (119) with the outer surface of the body region (111) on it, within any given cross-section of the glass syringe barrel (100) that is located in a plane (120) being centrically located in the glass syringe barrel (100) and comprising the longitudinal axis **L_{barrel}** of the glass syringe barrel (100), **f(x)** defines the vertical distance between the substrate (119) and the outer surface of the glass syringe barrel (100) at a given position **x,** $k \left(x\right) = \left|f"\left(x\right)/{\left[1+f′{\left(x\right)}^{2}\right]}^{3 / 2}\right|$ defines the absolute value of the curvature of **f(x)** at a given position **x,** and
in the interval between **x** = **P₁** and **x** = **P₂** for any concave curvature in this interval the minimum value for (1/**k(x)**)/**n₂**² is at least 0.5 mm⁻¹, wherein **P₁** defines the x-position at which the outer diameter of the glass syringe barrel (100) is 0.95 × **d₂** and **P₂** is **P₁** + 3 ×**n₂**

5. The plurality of glass syringe barrels (100) according to anyone of claims 1 to 4, wherein for at least 75 % of the glass syringe barrels (100) contained in the plurality of glass syringe barrels in the interval between **x** = **P₁** and **x** = **P₂** the maximum value of the first derivative **f'(x)ₘₐₓ** of **f(x)** is less than 18.

6. The plurality of glass syringe barrels (100) according to anyone of claims 1 to 5, wherein for at least 75 % of the glass syringe barrels (100) contained in the plurality of glass syringe barrels (100) **r₂** is in the range from 1.6 to 2.7 mm.

7. The plurality of glass syringe barrels (100) according to anyone of claims 1 to 6, wherein the glass syringe barrel (100) further comprises a constriction region (108) that is located between the cone region (105) and the shoulder region (111), the constriction region (108) having a first end (109) that is adjacent to the second end (107) of the cone region (105), a second end (110) that is adjacent to the first end (112) of shoulder region (111) and an outer contour **c₃**, wherein the constriction region (108) has a length **l₁**', a minimum outer diameter **d₁**' < **d₁** below the first end (109) of the constriction region (108) and an outer diameter **d₁**" at the second end (110) of the constriction region (108).

8. The plurality of glass syringe barrels (100) according to claim 7, wherein the outer contour **c3** of the glass syringe barrel (100) in the constriction region (108) is conically shaped with **d₁' < d₁"** and wherein at the second end (110) of the constriction region (108) **c₃** merges into **c₁** without any offset.

9. The plurality of glass syringe barrels (100) according to claim 8, wherein in the constriction region (108) a first line (117) that runs parallel to the longitudinal axis **L_{barrel}** and a second line (118) that runs parallel to **c₃** and that runs in the same plane as the first line (117) include an angle **γ,** wherein for at least 75 % of the glass syringe barrels (100) contained in the plurality of glass syringe barrels (100) **γ** is in the range from 0.3 to 2.5°.

10. The plurality of glass syringe barrels (100) according to anyone of claims 1 to 9, wherein for at least 75 % of the glass syringe barrels (100) contained in the plurality of glass syringe barrels (100) **l₁** is in the range from 8 to 12 mm.

11. The plurality of glass syringe barrels (100) according to anyone of claims 7 to 9, wherein for at least 75 % of the glass syringe barrels (100) contained in the plurality of glass syringe barrels (100) **l₁**' is in the range from 1 to 3 mm.

12. The plurality of glass syringe barrels (100) according to anyone of claims 1 to 11, wherein in at least 75 % of the glass syringe barrels (100) contained in the plurality of glass syringe barrels (100) the shoulder region (111) is **characterized by** an outer shoulder angle **α** in the range from 6 to 28 °.

13. The plurality of glass syringe barrels (100) according to anyone of claims 1 to 12, wherein at least a part of the outer surface of the glass syringe barrel (100) in the cone region (105) is roughened or is provided with a coating.

14. The plurality of glass syringe barrels (100) according to anyone of claims 1 to 13, wherein the glass syringe barrel (100) is sealed with a tip cap (118) that is attached to the conically shaped upper portion (101).

15. A plurality of syringes including at least 100 syringes comprising
A) the plurality of glass syringe barrels (100) according to anyone of claims 1 to 14;
B) a plunger stopper (104) that has been pushed into the bottom end (103) of each of the glass syringe barrels (100) contained in the plurality of glass syringe barrels.

## Patentansprüche

1. Eine Vielzahl von Glasspritzenzylindern (100), die mindestens 100 Glasspritzenzylinder (100) umfasst, wobei jeder Glasspritzenzylinder (100) einen zumindest teilweise konisch geformten oberen Abschnitt (101) aufweist und jeder Glasspritzenzylinder (100) eine Längsachse **L_{Zylinder}** aufweist und ein oberes Ende (102), durch das eine Flüssigkeit ausgestoßen werden kann, sowie ein unteres Ende (103), in das ein Kolbenstopfen (104) eingeschoben werden kann, wobei jeder Glasspritzenzylinder (100) in einer Richtung vom oberen Ende (102) zum unteren Ende (103) Folgendes umfasst:
i) einen konischen Bereich (105) mit einem ersten Ende (106), das dem oberen Ende (102) des Glasspritzenzylinders (100) entspricht, und einem zweiten Ende (107), wobei der konische Bereich (105) eine Länge **l₁** und am zweiten Ende (107) einen Außendurchmesser **d₁** aufweist;
ii) einen Schulterbereich (111) mit einem ersten Ende (112), das an das zweite Ende (107) des Konusbereichs (105) angrenzt, und einem zweiten Ende (113), wobei die Außenkontur des Schulterbereichs (111) einen konkaven und im Wesentlichen kreisbogenförmigen Bereich **c₁** mit einem Außenradius **r_{(1),}**der unterhalb des zweiten Endes (107) des Kegelbereichs (105) beginnt, sowie einen konvexen und im Wesentlichen kreisbogenförmigen Bereich **c₂**mit einem Außenradius **r_{(2),}**der oberhalb des zweiten Endes (113) des Schulterbereichs (111) beginnt;
iii) einen Körperbereich (114) mit einem ersten Ende (115), das an das zweite Ende (113) des Schulterbereichs (111) angrenzt, und einem zweiten Ende (116), das dem unteren Ende (103) des Glasspritzenzylinders (100) entspricht, wobei der Körperbereich (114) einen Außendurchmesser **d₂** und eine Glasdicke **n₂** aufweist;
wobei für die Bruchfestigkeit des Luer-Konus das 1-%-Quantil der in der Vielzahl von Glasspritzenzylindern (100) enthaltenen Glasspritzenzylinder mindestens 95 N beträgt ;
wobei für mindestens 75 % der in der Vielzahl von Glasspritzenzylindern (100) enthaltenen Glasspritzenzylinder (100) **r₁** im Bereich von 0,5 bis 2,5 mm liegt;
wobei für mindestens 75 % der in der Vielzahl von Glasspritzenzylindern (100) enthaltenen Glasspritzenzylinder (100) **r₂** im Bereich von 1,4 bis 2,9 mm liegt.

2. Die Vielzahl von Glasspritzenzylindern (100) nach Anspruch 1, wobei die Vielzahl von Glasspritzenzylindern (100) mindestens 200 Glasspritzenzylinder (100) umfasst.

3. Die Vielzahl von Glas-Spritzenzylindern (100) nach Anspruch 1 oder 2, wobei bei mindestens 75 % der in der Vielzahl von Glas-Spritzenzylindern (100) enthaltenen Glas-Spritzenzylinder (100) **r₁** im Bereich von 0,6 bis 2 mm liegt.

4. Die Vielzahl von Glasspritzenzylindern (100) gemäß einem der Ansprüche 1 bis 3, wobei für mindestens 75 % der in der Vielzahl von Glasspritzenzylindern (100) enthaltenen Glasspritzenzylinder (100) die folgenden Bedingungen erfüllt sind:
Wenn der Glasspritzenzylinder (100) mit der Außenfläche des Körperbereichs (111) auf einem ebenen, horizontalen Substrat (119) platziert wird, definiert f(x) innerhalb eines beliebigen Querschnitts des Glasspritzenzylinders (100), der in einer Ebene (120) liegt, die zentrisch im Glasspritzenzylinder (100) angeordnet ist und die Längsachse **L_{Zylinder}** des Glasspritzenzylinders (100) liegt, den vertikalen Abstand zwischen dem Substrat (119) und der Außenfläche des Glasspritzenzylinders (100) an einer gegebenen Position **x,** $k \left(x\right) = \left|f"\left(x\right)/{\left[1+f′{\left(x\right)}^{2}\right]}^{3 / 2}\right|$ definiert den Absolutwert der Krümmung von **f(x)** an einer gegebenen Position **x**, und
im Intervall zwischen **x** = **P₁** und **x** = **P₂** gilt für jede konkave Krümmung in diesem Intervall, dass der Minimalwert für (1/**k(x)**)/**n₂**² mindestens 0,5 mm⁻¹ beträgt, wobei **P₁** die x-Position definiert, an der der Außendurchmesser des Glaszylinders (100) der Spritze 0,95× **d₂** beträgt**_{,}** und **P₂** gleich **P₁** + 3× **n_{2 ist}**.

5. Die Vielzahl von Glasspritzenzylindern (100) gemäß einem der Ansprüche 1 bis 4, wobei für mindestens 75 % der in der Vielzahl von Glasspritzenzylindern enthaltenen Glasspritzenzylinder (100) im Intervall zwischen **x** = **P₁** und **x** = **P₂** der Maximalwert der ersten Ableitung **f'(x)ₘₐₓ** von **f(x**) kleiner als 18 ist.

6. Die Vielzahl von Glasspritzenzylindern (100) gemäß einem der Ansprüche 1 bis 5, wobei bei mindestens 75 % der in der Vielzahl von Glasspritzenzylindern (100) enthaltenen Glasspritzenzylinder (100) **r₂** im Bereich von 1,6 bis 2,7 mm liegt.

7. Die Vielzahl von Glasspritzenzylindern (100) gemäß einem der Ansprüche 1 bis 6, wobei der Glasspritzenzylinder (100) ferner einen Verengungsbereich (108) umfasst, der sich zwischen dem Konusbereich (105) und dem Schulterbereich (111) befindet, wobei der Verengungsbereich (108) ein erstes Ende (109), das an das zweite Ende (107) des Konusbereichs (105) angrenzt, ein zweites Ende (110), das an das erste Ende (112) des Schulterbereichs (111) angrenzt, und eine Außenkontur **c₃** aufweist , wobei der Verengungsbereich (108) eine Länge **l₁^{'}** , einen minimalen Außendurchmesser **d₁'** < **d₁** unterhalb des ersten Endes (109) des Verengungsbereichs (108) und einen Außendurchmesser **d₁**'' am zweiten Ende (110) des Verengungsbereichs (108) aufweist.

8. Die Vielzahl von Glasspritzenzylindern (100) gemäß Anspruch 7, wobei die Außenkontur **c3** des Glasspritzenzylinders (100) im Verengungsbereich (108) konisch geformt ist mit **d₁ '** < **d₁ "** und wobei am zweiten Ende (110) des Verengungsbereichs (108) **c₃** ohne Versatz in **c₁** übergeht.

9. Die Vielzahl von Glasspritzenzylindern (100) nach Anspruch 8, wobei im Verengungsbereich (108) eine erste Linie (117), die parallel zur Längsachse **L_{Zylinder}** verläuft, und eine zweite Linie (118), die parallel zu **c₃** verläuft und in derselben Ebene wie die erste Linie (117) liegt, einen Winkel γ einschließen, wobei für mindestens 75 % der in der Vielzahl von Glasspritzenzylindern (100) enthaltenen Glasspritzenzylinder (100) γ im Bereich von 0,3 bis 2,5° liegt.

10. Die Vielzahl von Glasspritzenzylindern (100) gemäß einem der Ansprüche 1 bis 9, wobei für mindestens 75 % der in der Vielzahl von Glasspritzenzylindern (100) enthaltenen Glasspritzenzylinder (100) **l₁** im Bereich von 8 bis 12 mm liegt.

11. Die Vielzahl von Glasspritzenzylindern (100) gemäß einem der Ansprüche 7 bis 9, wobei für mindestens 75 % der in der Vielzahl von Glasspritzenzylindern (100) enthaltenen Glasspritzenzylinder (100) **l₁^{'}** im Bereich von 1 bis 3 mm liegt.

12. Die Vielzahl von Glasspritzenzylindern (100) gemäß einem der Ansprüche 1 bis 11, wobei bei mindestens 75 % der in der Vielzahl von Glasspritzenzylindern (100) enthaltenen Glasspritzenzylinder (100) der Schulterbereich (111) durch einen äußeren Schulterwinkel α im Bereich von 6 bis 28° gekennzeichnet ist.

13. Die Vielzahl von Glasspritzenzylindern (100) gemäß einem der Ansprüche 1 bis 12, wobei zumindest ein Teil der Außenfläche des Glasspritzenzylinders (100) im Konusbereich (105) aufgeraut oder mit einer Beschichtung versehen ist.

14. Die Vielzahl von Glasspritzenzylindern (100) gemäß einem der Ansprüche 1 bis 13, wobei der Glasspritzenzylinder (100) mit einer Spitzenkappe (118) verschlossen ist, die an dem konisch geformten oberen Abschnitt (101) angebracht ist.

15. Eine Vielzahl von Spritzen, die mindestens 100 Glasspritzenzylinder (100) umfasst, umfassend
A) die Vielzahl von Glasspritzenzylindern (100) gemäß einem der Ansprüche 1 bis 14;
B) einen Kolbenstopfen (104), der in das untere Ende (103) jedes der in der Vielzahl von Glasspritzenzylindern enthaltenen Glasspritzenzylinder (100) eingeschoben wurde.

## Revendications

1. Une pluralité de corps de seringues en verre (100) comprenant au moins 100 corps de seringues en verre (100), chaque corps de seringue en verre (100) présentant une partie supérieure (101) de forme au moins partiellement conique et chaque corps de seringue en verre (100) présentant un axe longitudinal **L_{cylindre}** et comportant une extrémité supérieure (102) par laquelle un liquide peut être éjecté et une extrémité inférieure (103) dans laquelle un bouchon-piston (104) peut être enfoncé, chaque corps de seringue en verre (100) comportant, dans une direction allant de l'extrémité supérieure (102) vers l'extrémité inférieure (103) :
i) une région conique (105) présentant une première extrémité (106) qui correspond à l'extrémité supérieure (102) du corps de seringue en verre (100) et une deuxième extrémité (107), la région conique (105) ayant une longueur **l₁** et un diamètre extérieur **d₁** au niveau de la deuxième extrémité (107) ;
ii) une zone d'épaulement (111) comportant une première extrémité (112) adjacente à la deuxième extrémité (107) de la zone conique (105) et une deuxième extrémité (113), le contour extérieur de la zone d'épaulement (111) comprenant une zone **c₁** concave et sensiblement en forme d'arc de cercle, de rayon extérieur **r₁** commençant en dessous de la deuxième extrémité (107) de la zone conique (105) et une zone **c₂**convexe et sensiblement en forme d'arc de cercle, avec un rayon extérieur **r_{(2),}**commençant au-dessus de la deuxième extrémité (113) de la zone d'épaulement (111) ;
iii) une zone de corps (114) présentant une première extrémité (115) adjacente à la deuxième extrémité (113) de la zone d'épaulement (111) et une deuxième extrémité (116) correspondant à l'extrémité inférieure (103) du corps de la seringue en verre (100), la zone de corps (114) présente un diamètre extérieur **d₂** et une épaisseur de verre **n₂** ;
dans laquelle, en ce qui concerne la résistance à la rupture du cône Luer, le quantile à 1 % des corps de seringues en verre compris dans La pluralité de corps de seringues en verre (100) est d'au moins 95 N ;
dans lequel, pour au moins 75 % des corps de seringues en verre (100) compris dans La pluralité de corps de seringues en verre (100), **r₁** est compris entre 0,5 et 2,5 mm ;
dans laquelle, pour au moins 75 % des corps de seringues en verre (100) contenus dans la pluralité de corps de seringues en verre (100), **r₂** est compris entre 1,4 et 2,9 mm.

2. La pluralité de corps de seringues en verre (100) selon la revendication 1, dans lequel La pluralité de corps de seringues en verre (100) comprend au moins 200 corps de seringues en verre (100).

3. La pluralité de corps de seringues en verre (100) selon la revendication 1 ou 2, dans lequel, pour au moins 75 % des corps de seringues en verre (100) compris dans La pluralité de corps de seringues en verre (100), **r₁** est compris entre 0,6 et 2 mm.

4. La pluralité de corps de seringues en verre (100) selon l'une quelconque des revendications 1 à 3, dans lequel, pour au moins 75 % des corps de seringues en verre (100) contenus dans La pluralité de corps de seringues en verre (100), les conditions suivantes sont remplies :
si le corps de seringue en verre (100) est placé sur un substrat plan horizontal (119) avec la surface extérieure de la région du corps (111) reposant sur celui-ci, dans toute section transversale donnée du corps de seringue en verre (100) située dans un plan (120) centré dans le corps de seringue en verre (100) et comprenant l'axe longitudinal **L_{cylindre}** du corps de seringue en verre (100), **f(x)** définit la distance verticale entre le substrat (119) et la surface extérieure du corps de seringue en verre (100) en une position donnée **x**, $k \left(x\right) = \left|f"\left(x\right)/{\left[1+f′{\left(x\right)}^{2}\right]}^{3 / 2}\right|$ définit la valeur absolue de la courbure de **f(x)** en une position donnée **x**, et
dans l'intervalle compris entre **x** = **P₁** et **x** = **P₍₂₎ ,**pour toute courbure concave dans cet intervalle, la valeur minimale de (1/**k(x)**)/**n₂**² est d'au moins 0,5 mm⁻¹ , où **P₁** définit la position x à laquelle le diamètre extérieur du corps de la seringue en verre (100) est de 0,95× **d₂** et **P₂** est égal à **P₁** + 3× **n₂** .

5. La pluralité de corps de seringues en verre (100) selon l'une quelconque des revendications 1 à 4, dans lequel, pour au moins 75 % des corps de seringues en verre (100) compris dans La pluralité de corps de seringues en verre, dans l'intervalle compris entre **x** = **P₁** et **x** = **P₂** , la valeur maximale de la dérivée première **f'(x)ₘₐₓ** de **f(x**) est inférieure à 18.

6. La pluralité de corps de seringues en verre (100) selon l'une quelconque des revendications 1 à 5, dans lequel, pour au moins 75 % des corps de seringues en verre (100) compris dans La pluralité de corps de seringues en verre (100), **r₂** est compris entre 1,6 et 2,7 mm.

7. La pluralité de corps de seringues en verre (100) selon l'une quelconque des revendications 1 à 6, dans lequel le corps de seringue en verre (100) comprend en outre une zone de rétrécissement (108) située entre la zone conique (105) et la zone d'épaulement (111), la zone de rétrécissement (108) présentant une première extrémité (109) adjacente à la deuxième extrémité (107) de la zone conique (105), une deuxième extrémité (110) adjacente à la première extrémité (112) de la zone d'épaulement (111) et un contour extérieur **c₃**, la zone de rétrécissement (108) présentant une longueur **l₁^{'}** , un diamètre extérieur minimal **d₁ '** < **d₁** en aval de la première extrémité (109) de la zone de rétrécissement (108) et un diamètre extérieur **d₁ "** au niveau de la deuxième extrémité (110) de la zone de rétrécissement (108).

8. La pluralité de corps de seringues en verre (100) selon la revendication 7, dans lequel le contour extérieur **c3** du corps de seringue en verre (100) dans la zone de rétrécissement (108) présente une forme conique avec **d₁ '** < **d₁ "** et dans laquelle, au niveau de la deuxième extrémité (110) de la zone de rétrécissement (108), **c₃** se fond dans **c₁** sans aucun décalage.

9. La pluralité de corps de seringues en verre (100) selon la revendication 8, dans lequel, dans la zone de rétrécissement (108), une première ligne (117) s'étendant parallèlement à l'axe longitudinal **L_{cylindre}** et une deuxième ligne (118) s'étendant parallèlement à **c₃** et dans le même plan que la première ligne (117) forment un angle γ , dans laquelle, pour au moins 75 % des corps de seringues en verre (100) compris dans La pluralité de corps de seringues en verre (100), γ est compris entre 0,3 et 2,5°.

10. La pluralité de corps de seringues en verre (100) selon l'une quelconque des revendications 1 à 9, dans laquelle, pour au moins 75 % des corps de seringues en verre (100) contenus dans la pluralité de corps de seringues en verre (100), **l₁** est compris entre 8 et 12 mm.

11. La pluralité de corps de seringues en verre (100) selon l'une quelconque des revendications 7 à 9, dans lequel, pour au moins 75 % des corps de seringues en verre (100) contenus dans La pluralité de corps de seringues en verre (100), **l₁^{'}** est compris entre 1 et 3 mm.

12. La pluralité de corps de seringues en verre (100) selon l'une quelconque des revendications 1 à 11, dans lequel, pour au moins 75 % des corps de seringues en verre (100) compris dans La pluralité de corps de seringues en verre (100), la zone d'épaulement (111) est **caractérisée par** un angle d'épaulement extérieur α compris entre 6 et 28°.

13. La pluralité de corps de seringues en verre (100) selon l'une quelconque des revendications 1 à 12, dans lequel au moins une partie de la surface extérieure du corps de seringue en verre (100) dans la zone conique (105) est rendue rugueuse ou est pourvue d'un revêtement.

14. La pluralité de corps de seringues en verre (100) selon l'une quelconque des revendications 1 à 13, dans lequel le corps de seringue en verre (100) est obturé par un capuchon d'embout (118) qui est fixé à la partie supérieure de forme conique (101).

15. Une pluralité de seringues comprenant au moins 100 corps de seringues en verre (100), comprenant
A) La pluralité de corps de seringues en verre (100) selon l'une quelconque des revendications 1 à 14 ;
B) un bouchon de piston (104) qui a été enfoncé dans l'extrémité inférieure (103) de chacun des corps de seringue en verre (100) contenus dans La pluralité de corps de seringue en verre.
